# Europäisches Patentamt
# European Patent Office
# Office européen des brevets

(19)

(11) Veröffentlichungsnummer: **0 129 668**
**B1**

(12) **EUROPÄISCHE PATENTSCHRIFT**

(45) Veröffentlichungstag der Patentschrift:
23.08.89

(21) Anmeldenummer: **84104706.1**

(22) Anmeldetag: **26.04.84**

(51) Int. Cl.⁴: **C 12 N 5/00,** A 01 H 1/02, A 01 G 7/00

(54) Vermehrungsverfahren für pflanzliche Zellverbände.

(30) Priorität: **29.04.83 CH 2317/83**

(43) Veröffentlichungstag der Anmeldung:
**02.01.85 Patentblatt 85/1**

(45) Bekanntmachung des Hinweises auf die Patenterteilung:
**23.08.89 Patentblatt 89/34**

(84) Benannte Vertragsstaaten:
**AT BE CH DE FR GB IT LI LU NL SE**

(56) Entgegenhaltungen:
**EP-A-0 107 141**
**DE-A-3 126 001**
**GB-A-1 095 758**
**GB-A-2 059 991**

**PLANT CELL REPORTS, Band 2, Nr. 4, 1982, Seiten 165-168, San Carlos, US; T.L. ADAMS: "A new procedure for increasing efficiency of protoplast plating and clone selection"**

(73) Patentinhaber: **CIBA- GEIGY AG, Klybeckstrasse 141, CH- 4002 Basel (CH)**

(72) Erfinder: **Shillito, Raymond Douglas, Dr., Kohlplatzweg 22, CH- 4310 Rheinfelden (CH)**
Erfinder: **Paszkowski, Jerzy, Dr., Oberdorfstrasse 5, CH- 4125 Riehen (CH)**
Erfinder: **Potrykus, Ingo, Dr., Im Stigler 54, CH- 4312 Magden (CH)**

(74) Vertreter: **Zumstein, Fritz, Dr., Bräuhausstrasse 4, D-8000 München 2 (DE)**

EP 0 129 668 B1

**Beschreibung**

Angesichts des raschen Anstiegs der Erdbevölkerung bildet die Züchtung von verwertbaren Pflanzen einen der Schwerpunkte biologischer Forschung.

Auf der einen Seite steht die Suche nach alternativen, reproduzierbaren Nahrungsmittel-, Energie- und Rohstoffquellen wie z. B. neuen Pflanzen-, insbesondere Hybridsorten mit vorteilhaften Eigenschaften, beispielsweise einer erhöhten Resistenz gegenüber Krankheitserregern (wie phytopathogenen Insekten, Pilzen, Bakterien, Viren etc.), gegenüber Witterungs- oder Standorteinflüssen (wie Hitze, Kälte, Wind, Bodenbeschaffenheit, Feuchtigkeit, Trockenheit etc.) oder mit einer gesteigerten Reserve- und Vorratsstoffbildung in Blättern, Samen, Knollen, Wurzeln, Stengeln etc. Andererseits steigt neben der Nachfrage an wertvoller Biomasse auch der Bedarf an pharmazeutisch verwendbaren, pflanzlichen Wirksubstanzen und deren Abkömmlingen, wie z. B. Alkaloiden, Steroiden usw., die auf Grund ihrer geringen Ausbeuten aus natürlichen Vorkommen in zunehmendem Masse auf alternativem Weg, beispielsweise durch Extraktion aus genmanipulierten Pflanzensorten zugänglich gemacht werden.

Es besteht daher ein steigendes Interesse an einer praktischen Möglichkeit der gezielten Manipulation zahlreicher Pflanzensorten.

Wie hinlänglich bekannt ist, können isolierte, totipotente Zellen höherer Pflanzen in oder auf polymerhaltigen Nährböden zur Ausbildung embryonaler Zellverbände und in einigen Fällen zur Entstehung lebens- und vermehrungsfähiger Pflanzen veranlasst werden. Da die relativ festen Zellwände jedoch im allgemeinen eine fast unüberbrückbare Barriere für Manipulationen wie Zellfusionen oder Gentransplantationen bilden, verwendet man in diesen Fällen vorteilhafterweise die nackten Protoplasten, die durch Entfernung der Zellwände nach an sich bekannten Methoden, mit Hilfe von Enzymen (Pektinasen, Cellulasen etc.) aus den entsprechenden totipotenten, intakten Zellen erhalten werden.

Als Geliermittel (gelling agent) für Nährmedien für Pflanzenzellen und insbesondere auch für Protoplasten wird bisher üblicherweise Agar eingesetzt. Dabei ist jedoch festzustellen, dass Agar auf die Protoplasten der meisten Arten toxisch wirkt und nur wenige exceptionelle Fälle bekannt sind, in denen sich besonders widerstandsfähige Protoplasten auch in Agar erfolgreich kultivieren lassen.

In jüngster Zeit begann daher die Suche nach anderen, vorteilhaften Nährmedien. Man stellte unter anderem fest, dass Alginat und Agar gleichwertige Plattierungseigenschaften besitzen [Adaoha-Mbanaso EN, Roscoe DH (1982) Plant Sci. Lett. 25: 61 - 66].

Agarose wurde bisher ausschliesslich zur Züchtung tierischer Zellen und zur Vermehrung von Mikroorganismen eingesetzt. Bei der Züchtung von pflanzlichen Zellen wird man aber wegen der völlig unterschiedlichen Physiologie mit einem ganz anders gelagerten Problemkreis konfrontiert. Methoden der tierischen Zellvermehrung sind zwangsläufig auf die Vermehrung von Pflanzenzellen nicht übertragbar, ebensowenig wie auf die Erzeugung von pflanzlichen Zellverbänden aus Protoplasten.

Der Erfindung lag somit die Aufgabe zugrunde, die bei der Verwendung von Agar oder gereinigtem Agar im Nährmedium bei der Züchtung von aus pflanzlichen Protoplasten gewonnenen Zellkulturen auftretenden Schwierigkeiten zu vermeiden und ein allgemeines, auf alle, nicht nur besonders robuste Protoplastenarten anwendbares Verfahren bereitzustellen, nach dem die Züchtung von Zellverbänden bzw. embryonalen Pflanzen unter Vermeidung der phytotoxischen Einflüsse des Nährmediums in einfacher, praxisgerechter Weise ermöglicht wird. Diese Aufgabe wird durch die vorliegende Erfindung in überraschender Weise gelöst.

Agarose ist einer der Bestandteile des Agar. Käuflicher Agar besteht nämlich hauptsächlich aus einer Mischung von neutraler Agarose und ionischem Agaropektin mit einer Vielzahl gebundener Seitengruppen. Handelsübliche Agarose wird nach allgemein üblichen, kommerziellen Methoden aus Agar hergestellt. Im allgemeinen bleiben einige Seitengruppen erhalten und bestimmen im wesentlichen die physikochemischen Eigenschaften wie Gelbildung und Gelier- und Schmelztemperatur.

Bei niedrigen Temperaturen schmelzende und gelierende Agarose erzeugt man z. B. durch das nachträgliche Einführen von Hydroxyethylgruppen in die Agarosemoleküle. Diese derart modifizierte Agarose soll hier und im folgenden als LMT-Agarose (low melting agarose) bezeichnet werden.

Es wurde nunmehr überraschend gefunden, dass die bei der Vermehrung von aus Protoplasten gebildeten Pflanzenzellen in Agar auftretenden Schwierigkeiten, insbesondere die hohe Sterblichkeit der Protoplasten, durch den Ersatz von Agar gegen Agarose ganz wesentlich reduziert oder sogar gänzlich überwunden werden. Die Vermehrung der aus Protoplasten gewonnenen Zellen kann dann noch weiter gesteigert werden, wenn man nicht nur ein durch Agarose, insbesondere LMT-Agarose, gefestigtes Nährmedium verwendet, sondern zusätzlich dieses mit den Protoplasten beimpfte Nährmedium in kleinere Segmente unterteilt und diese Segmente einzeln oder in Gruppen in einer Nährlösung bis zur Bildung von Zellverbänden gewünschter Grösse inkubiert.

Es war in keiner Weise voraussehbar, dass der Einsatz von Agarose beliebiger Herkunft die Fähigkeit zur Bildung von Zellkulturen aus den unterschiedlichen Protoplastensorten, insbesondere aus empfindlichen Protoplastensorten, entscheidend zu steigern vermag, so dass bisher nicht kultivierbare Protoplastensorten nunmehr ebenfalls zur Bildung von Zellverbänden bis hin zu ganzen Pflanzen angeregt werden können.

Die vorliegende Erfindung betrifft daher ein Verfahren zur Erzeugung proliferierender pflanzlicher Zellverbände, dadurch gekennzeichnet, dass man (a) isolierte Protoplasten oder isolierte, aus Protoplasten regenerierte Zellen in oder auf einem mit Agarose gelierten Nährmedium gleichmässig verteilt, und/oder (b) dieses vorbehandelte und gelierte Nährmedium segmentiert, die Segmente in eine Nährlösung einbringt und

die Kultivierung in beiden Fällen bis zur gewünschten Grösse der Zellverbände fortsetzt.

Unter Zellverband sollen Kulturen, bestehend aus einer überschaubaren (zählbaren) Anzahl pflanzlicher Zellen bis zu fertigen Pflanzen verstanden werden.

Besonders bevorzugt ist ein Verfahren zur Erzeugung proliferierender pflanzlicher Zellverbände, dadurch gekennzeichnet, dass man (a) isolierte Protoplasten oder isolierte, aus Protoplasten regenerierte Zellen in oder auf einem durch Agarose gelierten Nährmedium gleichmässig verteilt, oder (b) dieses vorbehandelte und gelierte Nährmedium segmentiert, die Segmente in das 1- bis 10000-fache, vorzugsweise 5-bis 10000-fache, insbesondere 20- bis 100-fache ihres Volumens einer zur Vermehrung pflanzlicher Zellen geeigneten Nährlösung einbringt und die Kultivierung in beiden Fällen bei Temperaturen zwischen 0° und 40°C bis zur gewünschten Grösse der Zellverbände fortsetzt.

Besonders vorteilhafte Temperaturen für die Kultivierung der Zellverbände liegen zwischen 12° und 30°C.

Im Vordergrund des Interesses steht die technisch-gewerbliche Züchtung von Pflanzen aus Protoplasten.

Bei dem neuen Verfahren geht man zweckmässigerweise so vor, dass man die für die Vermehrung von Mikroorganismen übliche Plattenkulturmethode derart modifiziert, dass man die auf bekannte Weise isolierten, sterilisierten und gereinigten Protoplasten, statt in Agar, in ein mit Agarose gefestigtes (Nähr)medium aufnimmt, sie darin gleichmässig dispergiert, und das auf diese Weise mit den Protoplasten beimpfte und mit Agarose gelierte Medium in kleinere, vorteilhafterweise gleichartige Segmente aufteilt, diese Segmente einzeln oder gemeinsam in einen mit geeigneter Nährlösung teilweise gefüllten Behälter gibt und diesen bei den genannten Temperaturen gegebenenfalls im Dunkeln gleichmässigen Schüttelbewegungen so lange aussetzt, bis Zellverbände oder embryonale, lebensfähige Pflänzchen gewünschter Grösse entstehen.

Die mit Zellverbänden durchsetzten Agarose-Segmente eignen sich in hervorragender Weise zur grosstechnischen Weiterkultivierung in Bioreaktoren für die Isolierung von wertvollen Inhaltsstoffen.

Embryonale Pflänzchen lassen sich zu adulten Pflanzen, z. B. Hybridpflanzen mit besonders vorteilhaften Eigenschaften, heranziehen und auf biologisch normalem Wege weitervermehren.

Der Ausdruck "Segment" steht hier stellvertretend für ein dreidimensionales üblicherweise gleichgrosses Gebilde mit unregelmässiger oder bevorzugt regelmässiger räumlicher Ausdehnung, z. B. für Scheiben, Kugeln, Kuben, Prismen, Säulen, Kegel usw. mit einem mittleren Querschnitt von 1 bis ca. 100 mm, vorzugsweise 2 bis ca. 60 mm, insbesondere 2 bis 10 mm. Für die grosstechnische Weiterkultivierung von Zellverbänden in Fermentern eignen sich insbesondere kugelförmige Segmente eines mittleren Durchmessers.

Das erfinderische Verfahren ist wirksamer als sämtliche bekannte Verfahren, insbesondere bei der Erzeugung von Zellkulturen aus empfindlichen Problem-Protoplasten. Es zeichnet sich durch einfache Verfahrensmassnahmen aus und kann auf Protoplasten unterschiedlicher Herkunft und auf fusionierte sowie genmanipulierte Protoplasten angewandt werden. Es eignet sich zur selektiven Züchtung von Pflanzenzellkulturen mit einer oder mehreren eingangs beschriebenen, vorteilhaften Eigenschaften. Es kann überdies auch auf aus Tumorgewebe gewonnene Protoplasten angewandt werden.

Die Verwendung von Agarose zur Züchtung von Pflanzenzellen bzw. Zellkolonien ist somit ein wesentlicher Bestandteil dieser Erfindung, dabei ist es gleichgültig, welche Agarose-Sorte man einsetzt, da die verschiedenen Agarose-Typen sich im Hinblick auf ihre erfindungsgemässe Verwendung nur graduell unterscheiden und im Verhältnis zu Agar prinzipiell besser zur Kultivierung von aus Protoplasten gebildeten pflanzlichen Zellverbänden verwenden lassen.

Obwohl bereits die Versuchsresultate aus den nachstehenden Beispielen 1 bis 5 zweifelsfrei zeigen, dass Agarose dem Agar bei der Kultivierung von Pflanzenzellen aus Protoplasten in allen Fällen weitaus überlegen ist, tritt in der Praxis auch bei der Verwendung von Agarose ein gewisses Problem auf. In einigen Fällen kann man nämlich beobachten, dass die in der Entstehung begriffenen Zellverbände in einer fortgeschrittenen Entwicklungsphase einen Wachstumsstillstand erfahren oder sogar absterben: Ersteres ist möglicherweise auf eine Art Erschöpfung oder ein Auslaugen des Nährmediums und letzteres auf die Zunahme toxischer Ausscheidungsprodukte der wachsenden Zellverbände zurückzuführen. Beide unerwünschte Effekte können jedoch überraschenderweise durch eine einfache Massnahme beseitigt werden. Teilt man nämlich das durch Agarose gefestigte und mit Protoplasten geimpfte Primärmedium in kleinere, vorteilhafterweise gleichgrosse bzw. gleichgeformte Segmente und umspült diese Segmente z. B. in einem Schüttelbehälter mit einem vorteilhafterweise grösseren Volumen einer geeigneten flüssigen Nährlösung, so beobachtet man ein ungehindertes Wachstum der Zellverbände. Dabei ist es unter Umständen vorteilhaft, wenn man je nach Protoplastensorte die Segmentierung des mit Agarose gefestigten Nährmedium 0 bis 7 Tage, in den meisten Fällen 3 bis 4 Tage, nach der Impfung mit den Protoplasten durchführt. Als bester Zeitpunkt für Petunia-Protoplasten erweist sich der 4. Tag, für Tabak und Crepis capillaris der 3. Tag nach der Impfung. Bei Brassica rapa-Protoplasten erfolgt die Segmentierung am besten sofort nach der Gelierung der Agarose.

Neben dem Einsatz von Agarose zur Verfestigung des Nährmediums stellt die Aufteilung des mit Agarose gefestigten und mit Protoplasten beimpften Nährmediums in geeignete Segmente einen wichtigen Bestandteil dieser Erfindung dar. Zu dieser Erfindung gehören auch die nach dem beschriebenen Verfahren erzeugten Zellverbände bzw. Pflanzen.

Die im Rahmen des beschriebenen Verfahrens eingesetzten Nährlösungen können zur Beschleunigung des Zellwachstums auch Spuren organischer Verbindungen, wie z. B. Vitamine, Kohlenstofflieferanten, wie Rohrzucker und Glukose, Phytohormone, wie Auxin und Cytokinin, und natürliche Extrakte wie Malzextrakte oder Kokosnussmilch und erforderlichenfalls sonstige zweckdienliche Zusätze enthalten. Die Züchtungs- bzw. Inkubationsbedingungen, z. B. Temperatur des Nährmediums, Lichteinwirkung und Dauer der Inkubation bzw.

Weiterzüchtung lassen sich den jeweiligen Gegebenheiten, wie Pflanzen- bzw. Protoplastensorte oder Grösse des Versuchs anpassen.

Die Gewinnung brauchbarer Protoplasten wird nachfolgend in den Vorschriften (a) bis (c) ausführlich beschrieben, dabei kann die zugrundeliegende Zellkultur einem beliebigen Organ oder Gewebe der Pflanze, z. B. den Wurzeln, Stengeln, Blättern, Blüten, Samen, Pollen etc., z. B. auch von Kallus-Kulturen entstammen. Die Methoden zur Erzeugung von Protoplasten sind allgemein bekannt und nicht auf die in den Vorschriften (a) bis (c) angegebenen Spezies beschränkt.

**Herstellungsvorschriften für die Gewinnung reiner Protoplasten**

<u>Vorschrift (a)</u>: Eine auf an sich bekannte Weise hergestellte Suspension von Zellkulturen auxotropher Linien der Spezies <u>Hyoscyamus muticus</u> wird zur Gewinnung der Protoplasten mit einer Lösung von 4 % w/v Cellulase Onozuka R. 10 (Yakult Co. Ltd. Japan); 2 % w/v Driselase (Chemische Fabrik Schweizerhalle, Schweiz); in 0,25 M Sorbit, 0,025 M $CaCl_2 \cdot 2H_2O$; 0,5 % w/v 2(n-Morpholin)-ethansulfonsäure (= MES) mit einem pH-Wert von 5,2 behandelt. Die Inkubation erfolgt in der Dunkelheit über Nacht bei 26°C. Das Gemisch wird dann mit einem Stahlsieb (Porendurchmesser 100 µm) filtriert und das Filtrat mit demselben Volumen einer 0,6 M Saccharose-Lösung verdünnt. Das verdünnte Filtrat wird mit einer Lösung von 0,16 M $CaCl_2 \cdot 2 H_2O$ und 0,5 % w/v MES (pH 5,6) überschichtet und die gebildeten Protoplasten an der Grenzfläche beider Schichten eingesammelt, zweimal mit der zur Überschichtung verwendeten Lösung gewaschen und in einem gemäss Gebhardt et al. (1981) Planta 153: 81 - 89 hergestellten Medium B1 aufbewahrt. [Medium B1 wird weiter unten als Nährmedium A bezeichnet].

<u>Vorschrift (b)</u>: Protoplasten der <u>Nicotiana tabacum</u> Linie VR2 werden gemäss Shillito et al. (1981) Mutat. Res. 81: 165 - 175 isoliert. Die Reinigung dieser Protoplasten wird jedoch derart modifiziert, dass man nach der Inkubation in der enzymatischen Lösung diese mit einem halben Volumen einer 0,6 M Saccharoselösung verdünnt, das verdünnte Gemisch wie bei Beispiel (a) mit der genannten Calciumchlorid-Lösung überschichtet, an der Grenzfläche die Protoplasten entnimmt, mit einer gleichen Calciumchlorid-Lösung wäscht und danach wie in Vorschrift (a) verfährt und die Protoplasten im Medium K3 (unten als Nährmedium B bezeichnet) kultiviert.

<u>Vorschrift (c)</u>: Isolierung von Protoplasten zweier <u>Petunia hybrida</u> Linien des Haploid "Mitchell" (PMB Newsletter, 1980), welches M. Hanson, Charlottesville, VA. USA zur Verfügung stellte und der Mutator-Gen Petunia (Potrykus I (1970) Z. Pflanzenzüchtung 63: 24 - 40). Hierbei werden junge, voll entwickelte Blätter 10 Minuten mit einer Lösung von 0,01 % w/v $HgCl_2$ und 3 Tropfen Tween 80 auf 100 ml sterilisiert und fünfmal mit destilliertem Wasser gewaschen. Jeweils 6 Blatthälften ohne Mittelader werden aufeinander gelegt und mit einer osmotischen Lösung (0,375 M Mannit, 0,05 M $CaCl_2$ 0,5 % w/v MES; pH 5,8) benetzt und in dünne, 0,5 mm breite Streifen geschnitten. In diese Streifen wird im Vakuum die Enzym-Lösung (0,2 w/v Cellulose Onozuka R. 10, 0,2 % w/v Macerozyme; pH 5,6 in der Osmoselösung) infiltriert. Die Inkubation erfolgt über Nacht bei 12°C in der Dunkelheit. Anschliessend wird 1 Volumen der obigen osmotischen Lösung zugefügt und das Gemisch durch ein Sieb mit 100 µm Porenweite gefiltert. Die Protoplasten werden zweimal mit der osmotischen Lösung gewaschen und zur Beseitigung unerwünschter Abfallprodukte mit 0,6 M Saccharose-Lösung unterschichtet. Die an der Grenzfläche entnommenen Protoplasten werden noch einmal mit der osmotischen Lösung gereinigt und in einem Kultur-Medium [nach: Durand et al. (1973), Z. Pflanzenphysiol. 69: 26 - 34] für 12 Stunden in der Dunkelheit und bei 26°C inkubiert.

Die nachfolgend aufgeführten Prüfresultate der vergleichenden Untersuchungen veranschaulichen überzeugend die überraschenden, vorteilhaften Auswirkungen, die a) durch Ersatz des Agars durch Agarose und b) durch die Segmentierung der durch Agarose gelierten Nährmedien bei der Züchtung von pflanzlichen Zellverbänden aus Protoplasten zu beobachten sind.

**Beispiele vergleichender Untersuchungen von Agar und Agarose als Nährmedium**

α) <u>Versuchsbedingungen</u>
Sämtliche Nährlösungen werden durch Ultrafiltration (Nalgene-Filter, Porenquerschnitt 0,22 µm) sterilisiert. Zur Herstellung gelierter Nährböden werden gleiche Volumina doppelt konzentrierter Nährlösung mit doppelt konzentrierten und autoklaviertem Geliermittel gemischt.

Als flüssige Nährlösungen eignen sich z. B. die nachfolgend mit A, B, C, D und E benannten Kulturmedien bzw. Nährlösungen:

A) Complete medium B1: Gebhardt et al. Planta, 153, 81 - 89, (1981).
B) K3 medium: Nagy und Maliga, Z. Pflanzenphysiologie 78, 453 - 455 (1976).
C) DPD medium: Durand et al. Z. Pflanzenphysiologie 69, 26 - 34 (1973).

D) gemäss: Lindsmaier, E.M. und Skoog, F., Physiologia Plantarum 18, 100 - 127 (1965).
E) gemäss: Nitsch, J.P. und Nitsch, C., Science 163, 85 - 87 (1969).

Als Gelier- oder Eindickungsmittel werden verwendet:

(1) Agarose: in allen Fällen Sea Plaque LMT® (Marine Colloids). [Nur in den, in dem nachfolgend aufgeführten Beispiel 5 referierten Versuchen werden weitere, dort genannte Agarosesorten eingesetzt].
(2) Agar: In allen Versuchen Difco-Bacto-Agar.
(3) Gereinigter Agar: 454 g Agar [gemäss (2)] wird nacheinander mit 10 Liter Wasser, 5 Liter Aceton und 5 Liter Ethanol gewaschen und im Vakuum bei 40°C getrocknet. Es resultiert ein weisses, geruchloses Pulver.

Die gemäss den Vorschriften (a) bis (c) erhaltenen Protoplasten werden nach der üblichen Plattenkulturmethode in Petrischalen in dünnen Schichten in die gelierenden und gereinigten Nährmedien eingebracht. Der Agar bzw. die Agarose werden, sofern nicht speziell genannt, in einer Konzentration von 0,4 % w/v eingesetzt. Es werden jeweils 3 ml des Mediums in Petrischalen mit 6 cm Durchmesser und 10 ml des Mediums in Schalen mit 9 cm Durchmesser verwendet.

Bei Tests, in denen Agar- oder Agarose-Segmente Verwendung finden, werden die Protoplasten vor der Segmentierung des Mediums in letzterem gleichmässig verteilt. Die Segmente werden anschliessend in 30 ml der Nährlösung in Behältern mit 10 cm Durchmesser gegeben und auf einem Rotationsschüttler bei 26°C im Dunkeln oder bei Licht inkubiert und das flüssige Nährmedium in regelmässigen Intervallen oder kontinuierlich ausgetauscht.

Die Bewertung der Brauchbarkeit der Methode bzw. der günstigen Beeinflussung der Bildung von Zellkolonien aus Protoplasten erfolgt unter dem Mikroskop jeweils nach 4- bis 6-wöchiger Inkubationszeit durch Auszählen und Begutachten der gebildeten Zellverbände auf einer repräsentativen Anzahl von Rasterfeldern. In den Fällen der Segmentierungsversuche werden die Schalen mit den in der Nährlösung schwimmenden Segmenten in 14-tägigen Abständen photographiert und die in den Segmenten sichtbaren Zellverbände in Zahl und Grösse ausgewertet.

β) Testresultate

Beispiel 1: Anzahl der gebildeten Zellkolonien, entstanden nach Plattierung von $10^5$ Zellen oder $10^5$ Protoplasten der Spezies Hyoscyamus muticus, Linie VIII B9 (trp-) in 10 ml des gefestigten Nährmediums A)

| Gefestigtes Nährmedium A) | Zellen | Protoplasten |
|---|---|---|
| 0,4 % Agar | 20 | 0 |
| 0,4 % Agarose | nicht geprüft | 1102 |
| 0,8 % Agarose | 1024 | 472 |
| ohne Geliermittel | 1000 | 120 |

Die Resultate von Beispiel 1 zeigen, dass Agarose sowohl bei der Kultivierung von Zellen als auch von Zellen aus Protoplasten geeigneter ist als Agar und dass Agar auf die hier verwendeten Protoplasten und Zellen toxisch wirkt. Unter Dichte/ml soll hier und im folgenden die Zahl der Protoplasten bzw. Zellen pro ml Kulturmedium verstanden werden.

Beispiel 2: Wirksamkeit der Plattierung (%) von Tabak (VR2) Protoplasten bei steigender Verdünnung im Nährmedium B

| Dichte/ml: gefestigtes Nährmedium B) | 6000 | 3000 | 1000 | 300 |
|---|---|---|---|---|
| Agarose | Verband* | 4.3 | 2.2 | 0.22 |
| Agar | Verband* | 1.9 | 0 | 0 |
| ohne Geliermittel | Verband* | 1.3 | 0 | 0 |

* = Zellverband, dessen Dichte kein Auszählen zulässt

Wie aus den Resultaten des 2. Beispiels zu entnehmen ist, bilden Tabak (VR2) Protoplasten bei hoher Dichte bereits in Agar Zellverbände. Bei steigender Verdünnung überwiegt der positive Einfluss der Agarose auf die Entwicklung von Zellkolonien.

Ein beschleunigtes Wachstum bei kleinen Konzentrationen von Protoplasten wird auch durch die Resultate des dritten Beispiels für Protoplasten der Spezies Hyoscyamus muticus (VA5, his-) sowohl gegenüber gereinigtem Agar als auch gegenüber der flüssigen Phase (Nährlösung A) ohne Geliermittel bestätigt.

Beispiel 3: Wirksamkeit der Plattierung (%) von Hyoscyamus muticus (VA5, his-) Protoplasten bei steigender Verdünnung im Medium A

| Dichte/ml Gefestigtes Nährmedium A) | 11 000 | 3700 | 1300 | 600 |
|---|---|---|---|---|
| Agarose | Verband* | 9.5 | 3.2 | 0.25 |
| gereinigter Agar | 0.25 | 0.2 | 0 | 0 |
| ohne Geliermittel | 0.06 | 0 | 0 | 0 |

* = Zellverband, dessen Dichte kein Auszählen zulässt

Wie das nachfolgende Beispiel 4 anhand von Protoplasten der Spezies Hyoscyamus muticus (VIII B9 trp⁻) deutlich demonstriert, ist die toxische Wirkung des Agar mindestens teilweise auf Diffusionsvorgänge im Nährmedium zurückzuführen, da sowohl Agar als auch gereinigter Agar in der darübergeschichteten Agarose das Zellwachstum inhibieren.

Beispiel 4: Anzahl der gebildeten Zellkolonien, entstanden nach Plattierung von Protoplasten der Spezies Hyoscyamus muticus (VIII B9 trp⁻) in einer dünnen Schicht eines mit Agarose gelierten Nährmediums A unterschichtet mit Agarose oder gereinigtem Agar oder Agar oder in ungelierter Nährlösung (Gesamtvolumen 3 ml).

| Zahl der Protoplasten/ml | 50 000 | | 10 000 | |
|---|---|---|---|---|
| obere Schicht: Gefestigtes Nährmedium A) | identisch | Agarose | identisch | Agarose |
| Agarose | 215 | 221 | 66 | 68 |
| gereinigter Agar | 0 | 0 | 1 | 2 |
| Agar | 0 | 0 | 0 | 0 |
| ohne Geliermittel | 48* | | 3,3* | |

* flüssige Kontrolle

Das nachfolgende Beispiel 5 zeigt die prinzipielle Eignung unterschiedlicher Agarosearten bei der Kultivierung von Protoplasten der Spezies Hyoscyamus muticus und Tabak (VR2), die sich bei hoher und niedriger Protoplastendichte ergibt. Dabei rangieren gereinigter Agar und Agar am Ende der Liste, während Sea Plaque, BRL-LMT-Agarosen ähnliche Charakteristiken aufweisen und allen anderen geprüften Agarosearten deutlich überlegen sind. Gereinigter Agar und Agar zeigen gegenüber allen Agarosen und in allen Versuchen wesentlich schlechtere Ergebnisse und üben im allgemeinen eine eher entwicklungshemmende Wirkung auf Protoplasten aus.

Beispiel 5: Rangfolge der untersuchten Agar- und Agarosearten in der qualitativen Brauchbarkeit bei der Züchtung von Zellkulturen aus Protoplasten der Spezies Hyoscyamus muticus und Tabak (VR2)

| Rang | Agarose-Typ | Hersteller | |
|---|---|---|---|
| 1 | Sea Plaque LMT® | Marine Colloids | |
| | LMP® | Bethesda Research Laboratories (BRL) | |
| 3 | Typ VII® | Sigma | |
| 4 | HGT® | Sigma | geeignet |
| 6 | HGTP®, HGT®, LE® | Marine Colloids | |
| 8 | Standard LMT® | BioRoad | |
| 9 | Sea-Prep® (0,8 %)* | Marine Colloids | |
| 10 | gereinigter Agar | | für die Praxis unge- |
| 11 | Bacto-Agar | Difco | eignet, da oft toxisch |

* Alle Tests werden bei Konzentrationen von 0,8 % und 0,4 % w/v durchgeführt, da bei diesen Konzentrationen bereits ein Gel gebildet wird. Sea Prep® bildet erst über 0,8 % w/v Gele.

Der Einfluss auf die Bildung von Zellkulturen nach Segmentierung des gefestigten, mit Protoplasten beimpften Nährmediums, das sich in einer flüssigen Nährlösung befindet, wird anhand von Petunia-Protoplasten und zwar des Haploids "Mitchell" untersucht. Als Geliermittel für den der Segmente zugrundeliegenden Nährboden werden Agar, gereinigter Agar und Agarose eingesetzt. Man vergleicht dabei das Wachsen der Zellverbände in den Segmenten und in Platten gemäss der herkömmlichen Plattierungsmethode in Petri-Schalen. Wie die Test-Resultate des nachfolgenden Beispiels 6 zeigen, ist die Zahl der aus Protoplasten gebildeten Zellkolonien in den Segmenten stets grösser als in den herkömmlichen Platten ohne

EP 0 129 668 B1

umspülende Nährlösung. Unter allen untersuchten Medien führen Segmente aus Agarose zu dem vitalsten Wachstum der Zellverbände.

**Beispiel 6:** Vergleichende Untersuchung der Zellverbandentwicklung in gelierten Medien nach der herkömmlichen Plattentechnik und in segmentierter Form in einer Nährflüssigkeit.

Prozentualer Anteil der Zellkolonien des Petunia hybrida Haploids "Mitchell", die sich nach 2 Wochen in einem gefestigten Medium aus Protoplasten bilden und dabei entweder in Segmente unterteilt und in der Nährlösung* unter Schütteln aufbewahrt werden oder ungeteilt in den Petrischalen bleiben.

\* Nährlösung C)

| Dichte/ml: | $8 \times 10^4$ | | $4 \times 10^4$ | | $2 \times 10^4$ | | |
|---|---|---|---|---|---|---|---|
| Methode: | Platte | Segment | Platte | Segment | Platte | Segment | |
| Agarose | 14,8 | 22,8 | 26,0 | 31,2 | 27,2 | 52,4 | |
| gereinigter | | | | | | | [%] |
| Agar | 3,2 | 14,2 | 6,2 | 8,4 | 7,4 | 10,6 | |
| Agar | 2,6 | 5,4 | 2,2 | 3,2 | 0,12 | 0,81 | |

Der Versuch beweist einwändfrei die Überlegenheit der Verwendung von segmentierter Agarose in flüssigem Nährmedium gegenüber gereinigtem Agar und Agar sowohl in Plattenform, als auch in segmentierter Form.

Analoge Resultate erzielt man z. B. mit Protoplasten der Spezies Crepis capillaris, Protoplasten der Mutar-Gen Petunia hybrida "Potrykus", Protoplasten der Spezies Brassica rapa, der Spezies Lycopersicon esculentum, sowie der Spezies Nicotiana tabacum.

**Beispiel 7:** Regenerierung von Pflanzen aus Protoplasten der Spezies Nicotiana tabacum

Ca. $5 \times 10^4$ Protoplasten pro ml der Spezies Nicotiana tabacum werden in einem mit 1 % w/v Agarose gelierten Nährmedium B) [K3-medium] dispergiert und 10 Tage in geschlossenen Petrischalen in der Klimakammer (Licht 3000 Lux; Temperatur 24°C; Luftfeuchtigkeit ca. 100 %) kultiviert. Dann wird dieses gelierte Nährmedium in gleich grosse Segmente (Durchmesser 1 cm) geteilt und die Segmente in das 10-fache ihres Volumens einer modifizierten Nährlösung B) mit 0,25 Mol Saccharose, 0,05 mg/Liter 2,4-Dichlorphenoxyessigsäure, 2 mg/Liter 2-Naphthylessigsäure, 0,1 mg/Liter 6-Benzylaminopurin und 0,1 mg/Liter Kinetin eingebracht und darin gleichmässig geschüttelt. Nach einer Woche wird die Nährlösung B) erneuert, wobei der Gehalt an Saccharose auf 0,2 Mol reduziert wird. Das Segment/Nährlösungsgemisch wird weiter vier Wochen geschüttelt und die Nährlösung jede Woche erneuert. Anschliessend werden die gebildeten Zellkolonien auf ein mit Agarose gefestigtes Nährmedium D) mit 0,2 Mol Saccharose, 0,05 mg/Liter 2,4-Dichlorphenoxyessigsäure, 2 mg/Liter 2-Naphthylessigsäure, 0,1 mg/Liter 6-Benzylaminopurin und 0,1 mg/Liter Kinetin aufgetragen. Diese statische Kultur bildet innerhalb von vier Wochen Kalluskulturen, die auf ein mit Agarose gefestigtes Nährmedium D) mit 0,2 mg/Liter 6-Benzylaminopurin übertragen werden, wo sich einzelne Sprösslinge bilden. Letztere werden auf ein mit Agar gefestigtes Nährmedium E) übertragen und werden nach Ausbildung von Wurzeln in Erde verpflanzt und zu reifen Pflanzen herangezogen.

**Patentansprüche**

1. Verfahren zur Erzeugung proliferierender Zellverbände, dadurch gekennzeichnet, dass man

a) isolierte Protoplasten oder isolierte aus Protoplasten regenerierte Zellen in oder auf einem durch Agarose gelierten Nährmedium gleichmässig verteilt, und/oder

b) dieses vorbehandelte und gelierte Nährmedium segmentiert, die Segmente in eine Nährlösung einbringt und

die Kultivierung in beiden Fällen bis zur gewünschten Grösse der Zellverbände fortsetzt.

2. Verfahren nach Anspruch 1, dadurch gekennzeichnet, dass man a) isolierte Protoplasten oder isolierte aus Protoplasten regenerierte Zellen in oder auf einem durch Agarose gelierten Nährmedium gleichmässig verteilt, oder b) dieses vorbehandelte und gelierte Nährmedium segmentiert, die Segmente in das 1- bis 10000-fache ihres Volumens einer zur pflanzlichen Zellvermehrung geeigneten Nährlösung einbringt und die Kultivierung in beiden Fällen bei Temperaturen zwischen 0° und 40°C bis zur gewünschten Grösse der Zellverbände fortsetzt.

3. Verfahren nach einem der Ansprüche 1 oder 2, dadurch gekennzeichnet, dass man manipulierte Protoplasten oder manipulierte Protoplasten mit bereits wieder regenerierten Zellwänden einsetzt.

4. Verfahren nach Anspruch 3, dadurch gekennzeichnet, dass es sich bei der Manipulation um Gen-, Organellentransplantation oder Zellfusion handelt.

5. Verfahren nach einem der Ansprüche 1 bis 4, dadurch gekennzeichnet, dass man als Inkubationstemperatur den Bereich zwischen 12° und 30°C wählt.

6. Verfahren nach Anspruch 2, dadurch gekennzeichnet, dass man die Segmente in das 5- bis 10000-fache

7

ihres Volumens einer zur pflanzlichen Zellvermehrung geeigneten Nährlösung einbringt.

7. Verfahren nach Anspruch 6, dadurch gekennzeichnet, dass man die Segmente in das 20- bis 100-fache ihres Volumens einer zur pflanzlichen Zellvermehrung geeigneten Nährlösung einbringt.

8. Verfahren nach einem der Ansprüche 1 bis 7, dadurch gekennzeichnet, dass man die Segmentierung nach der Impfung des mit Agarose gefestigten (Primär)Nährmediums mit Protoplasten im Zeitraum von 0 bis 7 Tagen durchführt.

9. Verfahren nach Anspruch 8, dadurch gekennzeichnet, dass man die Segmentierung 3 bis 4 Tage nach der Impfung durchführt.

10. Verfahren nach einem der Ansprüche 1 bis 9, dadurch gekennzeichnet, dass man gleichgrosse Segmente regelmässiger räumlicher Ausdehnung mit einem mittleren Querschnitt von 1 bis ca. 100 mm einsetzt.

11. Verfahren nach Anspruch 10, dadurch gekennzeichnet, dass man Segmente mit einem mittleren Durchmesser von 2 bis 60 mm einsetzt.

12. Verfahren nach Anspruch 11, dadurch gekennzeichnet, dass man als Segmente Scheiben, Kugeln, Kuben, Prismen, Säulen oder Kegel mit einem mittleren Durchmesser von 2 bis 10 mm einsetzt.

13. Verfahren nach einem der Ansprüche 1 bis 12, dadurch gekennzeichnet, dass man Protoplasten einsetzt, die aus den Pflanzenspezies Nicotiana tabacum, Hyoscyamus muticus, Lycopersicon esculentum, Crepis capillaris, Brassica rapa oder Petunia hybrida isoliert werden.

14. Verfahren nach einem der Ansprüche 1 bis 13, dadurch gekennzeichnet, dass man Agarose der Typen Sea Plaque LMT®, LMP®, Typ VII®, HGT®, HGTP®, LE®, Standard LMT® oder Sea-Prep® verwendet.

15. Verfahren nach einem der Ansprüche 1 bis 14, dadurch gekennzeichnet, dass man die mit Protoplasten oder mit Protoplasten mit regenerierten Zellwänden geimpften und durch Agarose gefestigten Segmente grosstechnisch in Bioreaktoren einsetzt.

16. Verfahren nach einem der Ansprüche 1 bis 11, dadurch gekennzeichnet, dass man die Zellvermehrung bis zur Entwicklung der lebens- und vermehrungsfähigen Pflanzen durchführt.

17. Verwendung von Agarose als Vermehrungs- bzw. Kultivierungsmedium für Pflanzenzellkolonien aus Protoplasten oder Protoplasten mit bereits wieder regenerierten Zellwänden.

## Claims

1. A process for producing proliferating cell aggregates, which comprises

a) uniformly distributing isolated protoplasts or isolated cells regenerated from protoplasts in or on an agarose-solidified culture medium, and/or
b) cutting the pretreated and solidified culture medium into segments and transferring the segments to a nutrient solution,
and continuing culturing in both cases until the cell aggregates have attained the desired size.

2. A process according to claim 1, which comprises a) uniformly distributing isolated protoplasts or isolated cells regenerated from protoplasts in or on an agarose-solidified culture medium, or b) cutting the pretreated and solidified culture medium into segments, putting said segments into 1 to 10,000 times their own volume of a nutrient solution suitable for culturing plant cells, and continuing culturing in both cases in a temperature range of from 0° to 40°C until the cell aggregates have attained the desired size.

3. A process according to either of claims 1 and 2, wherein manipulated protoplasts or manipulated protoplasts with already regenerated cell walls are used.

4. A process according to claim 3, wherein the manipulation is a gene or organelle transplant or a cell fusion.

5. A process according to any one of claims 1 to 4, wherein the incubation temperature is in the range of from 12° to 30°C.

6. A process according to claim 2, wherein the segments are put into 5 to 10,000 times their own volume of a nutrient solution suitable for culturing plant cells.

7. A process according to claim 6, wherein the segments are put into 20 to 100 times their own volume of a nutrient solution suitable for culturing plant cells.

8. A process according to any one of claims 1 to 7, wherein the agarose-solidified primary culture medium is cut into segments after a period of 0 to 7 days after inoculation with protoplasts.

9. A process according to claim 8, wherein cutting into segments is effected 3 to 4 days after inoculation.

10. A process according to any one of claims 1 to 9, which comprises the use of segments of equal size and regular shape having an average diameter of 1 to about 100 mm.

11. A process according to claim 10, which comprises the use of segments having an average diameter of 2 to 60 mm.

12. A process according to claim 11, wherein the segments are discs, spheres, cubes, prisms, cylinders or cones having an average diameter of 2 to 10 mm.

13. A process according to any one of claims 1 to 12, which comprises the use of protoplasts isolated from the plant species Nicotiana tabacum, Hyoscyamus muticus, Lycopersicon esculentum, Crepis capillaris, brassica rapa or Petunia hybrida.

14. A process according to any one of claims 1 to 13, wherein agarose of the type Sea Plaque LMT®, LMP®,

type VII®, HGT®, HGTP®, LE®, Standard LMT®, or Sea Prep® is used.

15. A process according to any one of claims 1 to 14, wherein the agarose-solidified segments inoculated with protoplasts or with protoplasts with regenerated cell walls are used for large-scale culturing in bioreactors.

16. A process according to any one of claims 1 to 11, wherein cell culturing is carried out until plants that are viable and capable of propagation develop.

17. Use of agarose as a propagation or culture medium for forming plant cell colonies from protoplasts or from protoplasts with already regenerated cell walls.


**Revendications**

1. Procédé pour la production d'agrégats de cellules proliférants, caractérisé en ce que

a) l'on répartit régulièrement des protoplastes isolés ou des cellules isolées, régénérées à partir de protoplastes, dans ou sur un milieu nutritif gélifié par de l'agarose, et/ou
b) en ce que l'on segmente le milieu nutritif ainsi pré-traité et gélifié, en ce que l'on introduit les segments dans une solution nutritive et
en ce que l'on poursuit la culture, dans les deux cas, jusqu'à la grandeur désirée des agrégats de cellules.

2. Procédé selon la revendication 1, caractérisé a) en ce que l'on répartit régulièrement les protoplastes isolés ou les cellules isolées, régénérées à partir de protoplastes, dans ou sur un milieu nutritif gélifié par de l'agarose ou b) en ce que l'on segmente ce milieu nutritif pré-traité et gélifié, en ce que l'on introduit les segments dans 1 à 10.000 fois leur volume d'une solution nutritive appropriée pour la multiplication des cellules végétales et en ce que l'on poursuit la culture, dans les deux cas, à des températures entre 0° et 40°C jusqu'à la grandeur désirée des agrégats de cellules.

3. Procédé selon l'une des revendications 1 ou 2, caractérisé en ce que l'on utilise des protoplastes manipulés ou des protoplastes manipulés avec des membranes cellulaires déjà à nouveau régénérées.

4. Procédé selon la revendication 3, caractérisé en ce que la manipulation consiste en une transplatation de gènes, d'organites ou en une fusion de cellules.

5. Procédé selon la revendication 1, caractérisé en ce que l'on choisit comme température d'incubation l'intervalle compris entre 12° et 30°C.

6. Procédé selon la revendication 1, caractérisé en ce que l'on introduit les segments dans 5 à 10 000 fois leur volume d'une solution nutritive appropriée pour la multiplication des cellules végétales.

7. Procédé selon la revendication 6, caractérisé en ce que l'on introduit les segments dans 20 à 100 fois leur volume d'une solution nutritive appropriée pour la multiplication des cellules végétales.

8. Procédé selon l'une des revendications 1 à 7, caractérisé en ce que l'on effectue la segmentation dans un laps de temps de 0 à 7 jours après l'inoculation avec des protoplastes du milieu nutritif (primaire) consolidé par de l'agarose.

9. Procédé selon la revendication 8, caractérisé en ce que l'on effectue la segmentation 1 à 4 jours après l'inoculation.

10. Procédé selon l'une des revendications 1 à 9, caractérisé en ce que l'on utilise des segments identiques de dimensions régulières avec une section moyenne de 1 à environ 100 mm.

11. Procédé selon la revendication 10, caractérisé en ce que l'on utilise des segments avec un diamètre moyen de 2 à 60 mm.

12. Procédé selon la revendication 11, caractérisé en ce que l'on utilise comme segment des disques, des billes, des cubes, des prismes, des colonnes ou des cônes ayant un diamètre moyen de 2 à 10 mm.

13. Procédé selon l'une des revendications 1 à 12, caractérisé en ce que l'on utilise des protoplastes isolés des espèces végétales Nicotiana tabacum, Hyoscyamus muticus, Lycopersicon esculentum, Crepis capillaris, Brassica rapa ou Petunia hybrida.

14. Procédé selon l'une des revendications 1 à 13, caractérisé en ce que l'on utilise de l'agarose du type Sea Plaque LMT (marque déposée), LMP (marque déposée), Typ VII (marque déposée), HGT (marque déposée), HGTP (marque déposée), LE (marque déposée), Standard LMT (marque déposée) ou Sea-Prep (marque déposée).

15. Procédé selon l'une des revendications 1 à 14, caractérisé en ce que l'on utilise à l'échelle industrielle dans des bioréacteurs les segments inoculés avec des protoplastes ou avec des protoplastes avec des membranes cellulaires régénérées, et consolidés avec de l'agarose.

16. Procédé selon l'une des revendications 1 à 11, caractérisé en ce que l'on effectue la multiplication des cellules jusqu'au développement de plantes viables et aptes à se reproduire.

17. Utilisation de l'agarose comme milieu de multiplication et de culture pour des colonies de cellules végétales à partir de protoplastes ou de protoplastes avec des membranes cellulaires déjà à nouveau régénérées.